(19) Europäisches Patentamt
European Patent Office
Office européen des brevets

(11) Publication number: **0 438 182 A1**

# (12) EUROPEAN PATENT APPLICATION

(21) Application number: **91100682.3**

(22) Date of filing: **21.01.91**

(51) Int. Cl.5: **C12P 23/00**, C12N 1/14, C12R 1/645

The microorganism(s) has (have) been deposited with Agricultural Research Culture Collection under number NRRL Y-18579.

(30) Priority: **19.01.90 US 467607**

(43) Date of publication of application: **24.07.91 Bulletin 91/30**

(84) Designated Contracting States: **BE DE DK GB NL SE**

(71) Applicant: **PHILLIPS PETROLEUM COMPANY**
**5th and Keeler**
**Bartlesville Oklahoma 74004(US)**

(72) Inventor: **Prevatt, William Dudley**
**4524 Barlow Drive**
**Bartelsville, OK 74006(US)**
Inventor: **Dickson, Terry Donnell**
**Route 1, Box 53**
**Wann, OK 74083(US)**
Inventor: **Harris, Robin Lashell**
**1609 SE King Drive No. 724**
**Bartlesville, OK 74006(US)**

(74) Representative: **Dost, Wolfgang, Dr.rer.nat.,Dipl.-Chem. et al**
**Patent- & Rechtsanwälte Bardehle . Pagenberg . Dost . Altenburg . Frohwitter . Geissler & Partner Galileiplatz 1 Postfach 86 06 20**
**W-8000 München 86(DE)**

## (54) Novel strains of phaffia rhodozyma containing high levels of astaxanthin.

(57) The present invention provides novel strains of *Phaffia rhodozyma*, which produce high levels of astaxanthin.



Xerox Copy Centre

# NOVEL STRAINS OF *PHAFFIA RHODOZYMA* CONTAINING HIGH LEVELS OF ASTAXANTHIN

## Field of the Invention

The present invention relates to high productivity strains of *Phaffia rhodozyma* which produce high levels of astaxanthin.

## Background of the Invention

Astaxanthin (3,3'-dihydroxy-ß,ß-carotene-4,4'-dione) is a oxycarotenoid pigment widely distributed in plants and animals. It is a predominant oxycarotenoid pigment in crustaceans, and salmonids. Astaxanthin is also found in algae, yeast (such as *Phaffia rhodozyma*) and birds.

In commercial aquaculture it is desirable to add astaxanthin to the diet of salmonids and crustaceans to impart the distinctive pink coloration found in indigenous salmonids, crustaceans and birds. Imparting this distinctive pink coloration to salmonids and crustaceans produced by commercial aquaculture is believed to be important in encouraging consumer acceptance of salmonids and crustaceans produced through aquaculture. Currently no economical source for astaxanthin exists.

One potential source of astaxanthin for aquacultural purposes is the yeast in *Phaffia rhodozyma*. *Phaffia rhodozyma* has been recognized since its classification as a yeast species having a high astaxanthin content (~85% of its carotenoid pigment is astaxanthin, N.W. Miller, et al. Int. J. Syst. Bacteriol., Vol. 26, p. 286 (1976). Use of this yeast as a dietary supplement in salmonid and crustacean diets has also been explored by Eric A. Johnson and other researchers since the early 1980's.

The development of *Phaffia rhodozyma* as a commercial source of astaxanthin has been hampered by the absence of strains of *Phaffia rhodozyma* which produce high levels of astaxanthin. The strains of *Phaffia rhodozyma* currently available generally produce from 30 to 2000 micrograms per gram of cell mass. Although 2000 micrograms per gram of cell mass is a significant level of astaxanthin production it would be very advantageous to develop strains of *Phaffia rhodozyma* which produce higher levels of astaxanthin production per gram of cell mass (at least 2500 micrograms per gram of cell mass).

Thus it is an object of the present invention to provide strains of *Phaffia rhodozyma* which produce high levels of astaxanthin.

Other aspects, objects and several advantages of this invention will be apparent from the instant specification.

## Summary of the Invention

In accordance with the present invention, we have discovered and isolated novel strains of *Phaffia rhodozyma* which produce in the range of from about 2700 μg/g to about 8025 μg/g of astaxanthin when grown under suitable growth conditions in a shake flask.

## Detailed Description of the Invention

Wild strains of *Phaffia rhodozyma* can produce between 30-800 μg astaxanthin/g yeast, depending on the growth conditions (Johnson, E. A. and Lewis, M. J., 1979. J. Gen. Microbiol. 115:173-183). Improved strains such as those disclosed in WO 88/08025 can produce up to 2000 mg astaxanthin/g yeast under suitable growth conditions. While these levels of astaxanthin production are significant, to make commercial production of astaxanthin from *Phaffia rhodozyma* economically attractive, significant improvements in the level of astaxanthin production must be obtained.

The astaxanthin productivity in *Phaffia rhodozyma* strains can be improved by a systematic strain improvement program consisting of treating the yeast cells with a mutagen and then screening the resultant mutant cells able to produce colonies which have higher concentrations of astaxanthin. Mutagens suitable for this process include but are not limited to ultraviolet radiation, nitrous acid, ethylmethanesulfonate (EMS), diethyl sulfate, 1-methyl-1-nitro-nitrosoguanidine (nitrosoguanidine, NTG) and combinations of two or more thereof. These mutagens can be used to induce mutation in *Phaffia rhodozyma* following similar protocols to those developed for other yeast or bacteria, such as *Saccharomyces cerevisiae* or *Escherichia coli*. Currently preferred are yeast mutational protocols utilizing nitroguanadine or EMS. Suitable techniques for mutating yeasts such as *Phaffia rhodozyma*, are known to those skilled in the art including but not limited to the yeast mutation techniques discussed in Shermal, et al., Methods in Yeast Genetics,

Cold Spring Harbor Laboratory, 1979, or Example I sections A-C. Screening can be accomplished by visual inspection, astaxanthin extraction assays, and fermentation studies of those colonies appearing to have improved astaxanthin levels. Strains derived from colonies of mutagenized *Phaffia rhodozyma* should also be screened to ensure that the strains will retain desirable characteristics during and after repeated fermentations.

Many rounds of mutagensis and screening are necessary to develop *Phaffia rhodozyma* strains which have significantly improved astaxanthin production levels. Unfortunately each round of mutagensis independently produced a new group of mutants which have a progressively lower probability of providing improved strains of *Phaffia rhodozyma*. Additionally each round of mutagensis increased the likelihood that undesirable mutations would occur causing fewer of the strains to demonstrate improved astaxanthin production. After several rounds of mutation several strains which appeared to be high astaxanthin producers on plate screening turned out to produce low levels of astaxanthin in shake flasks. The following strains were mutated with nitrosoguanidine or ultraviolet light or combinations thereof, and demonstrated significantly improved astaxanthin production levels.

TABLE I

| Phillips Culture Collection No. | NRRL No. | Astaxanthin μg/g DCW[3] |
|---|---|---|
| PC 8055* | Y-10291 | 350 |
| PC 8089[4] | Y-18579 | 2727 |
| PC 8109[4] | Y-18581 | 5455 |
| PC 8108[4] | Y-18582 | 6578 |
| PC 8107[4] | Y-18583 | 8004 |

*The parent strain was 67-210, also known as PC 8055, which is deposited and accessible to the public from the United States Department of Agriculture, Agricultural Research Service, Northern Regional Research Center located in Peoria, Illinois under accession number NRRL Y-10291.

[1]Astaxanthin content was determined by the method described in Example I.C.2.

[2]The strains were grown under the conditions described in Example I.

[3]DCW is the abbreviation for dry cell weight. Example I.C. 2 explains the preferred procedure for determining DCW.

[4]The isolated substantially pure strains of *Phaffia rhodozyma* with corresponding NRRL numbers have been deposited with the United States Department of Agriculture, Agricultural Research Service, Northern Regional Research Center, 1815 North University Street, Peoria, Illinois 61604, under the terms of the Budapest Treaty.

With the inventive *Phaffia rhodozyma* strains PC 8089, PC 8109, PC 8108, and PC 8107 the increase in astaxanthin productivity is due to increased levels of astaxanthin production. The increased astaxanthin productivities of these strains under suitable growth conditions is in the range of from about 2700 μg/g to about 8025 μg/g of astaxanthin on a dry weight basis when cultivated in a shake flask. Preferably the astaxanthin productivity of these strains under the conditions described above will be in the range of from about 5500 μg/g to about 8025 μg/g on a dry weight basis and most preferably will range from about 6500 μg/g to about 8025 μg/g of astaxanthin on a dry weight basis. Suitable growth conditions in a shake flask are

defined as the conditions necessary to provide the maximum specific growth rate for the *Phaffia rhodozyma* strain being cultivated in a shake flask which is being vigorously agitated after 5 days of growth. Suitable growth conditions for the *Phaffia rhodozyma* strains of the present invention in a shake flask include utilizing Rich Assay Growth Medium as defined in the Examples of this application and cultivating the strain at between 20°C to 22°C with vigorous shaking.

Fermentation

*Phaffia rhodozyma* is a relatively new organism for use in industrial fermentation. Several workers in the area of *Phaffia rhodozyma* fermentation have observed that alcohol or aldehydes will accumulate in levels toxic to *Phaffia* if an excess carbon-energy source in the form of sugar is provided. This has led these workers to suggest growing *Phaffia rhodozyma* cells under conditions where the amount of carbon-energy source provided limits growth conditions. However, *Phaffia rhodozyma* responds to carbon-energy source limitation by producing lower astaxanthin yields and releasing compounds which cause excessive foaming in the fermentation vessel. The presence of these foam-causing compounds necessitates the use of antifoamants to avoid fermentation vessel overflow. Unfortunately the utilization of antifoamants can reduce the per cell astaxanthin yields.

We, however, have discovered that by maintaining a measurable excess of at least one suitable carbon-energy source in the fermentation broth containing the aqueous *Phaffia rhodozyma* cells and nutrients that alcohol and aldehyde production can be easily controlled and foaming avoided. Additionally the presence of a measurable excess of carbon-energy source also results in increased cell growth rates and astaxanthin yields.

Particularly important in improving astaxanthin and cell yields is the maintenance of a measurable excess of at least one suitable carbon-energy source while the *Phaffia rhodozyma* cells are in the transition phase between inoculation and the logarithmic growth phase. Preferably the *Phaffia rhodozyma* cells will be contacted with a measurable excess of at least one suitable carbon-energy source from the transition phase after inoculation through a substantial portion of the logarithmic growth phase.

The measurable excess of at least one suitable carbon-energy source provided should be an effective amount to avoid excessive foam formation during the fermentation of *Phaffia rhodozyma* and also not result in the generation of growth repressing or toxic levels of alcohol or aldehyde. Preferably the measurable excess of at least one carbon-energy source detectable in the fermentation broth consisting of the aqueous *Phaffia rhodozyma* cells and nutrients, will range from about 1.0 gram/liter to about 20 grams/liter and most preferably it will range from about 1.0 grams/liter to about 5.0 grams/liter. The amount of measurable excess of at least one suitable carbon-energy source in the fermentation broth should be controlled to avoid excess alcohol or aldehyde production. Preferably the amount of alcohol in the fermentation broth should range from about 0.0 grams/liter to about 3.0 grams/liter. Preferably the amount of aldehyde present in the fermentation broth will range from about 0.0 grams/liter to about 0.1 grams/liter.

The fermentation of *Phaffia rhodozyma* can be conducted in a aqueous continuous or batch-fed manner, utilizing a variety of carbon-energy sources and/or nutrient sources. Suitable carbon-energy sources for growing *Phaffia rhodozyma* include but are not limited to the carbon-energy source selected from the group consisting of succinate, furamate, malate, pyruvate, glucose, sucrose, fructose, maltose, corn syrup, hydrolyzed starch and combinations of any two or more thereof. Preferred carbon-energy sources for growing *Phaffia rhodozyma* are carbon-energy sources selected from the group consisting of succinate, glucose, and combinations thereof. A suitable nutrient or media source for *Phaffia rhodozyma* would include at least one nitrogen source, at least one phosphate source, at least one source of minerals such as iron, copper, zinc, magnesium, manganese, calcium, and other trace elements, and vitamins (such as biotin, pantothenic acid and thiamine) as required.

Suitable sources of at least one carbon-energy source and nutrients can be obtained from a variety of sources or may consist of a single source such as cane molasses. However, preferred are at least one carbon-energy source and/or nutrient sources which have a defined character. At least one carbon-energy source and nutrient composition which has proven particularly effective is set forth in Table 2.

TABLE 2

Carbon-Energy Source and Nutrients

| Component per Liter of Water | |
|---|---|
| Carbon-energy Source | 10 - 100 (g/l) |
| $H_3PO_4$ (85%) | 0.16 - 2.7 (ml/l) |
| $CaSO_4 \cdot 2H_2O$ | 0.011 - 0.8 (g/l) |
| $K_2SO_4$ | 0.171 - 1.3 (g/l) |
| $MgSO_4 \cdot 7H_2O$ | 0.140 - 1.56 (g/l) |
| KOH | 0.047 - 0.35 (g/l) |
| Biotin | 0.006 - 0.044 (mg/l) |
| Thiamine | 0.12 - 9.8 (mg/l) |
| [1]Yeast extracts | 1.2 - 6.0 (g/l) |
| [2]Minerals and Trace metals | 0.118 - 9.8 (ml/l) |

[1]Yeast extract is Amberex 1003 which is available from and a trademark of Universal Foods Corporation, Milwaukee, Wisconsin.

[2]Minerals and trace metals are $FeSO_4 \cdot 7H_2O$ 65.0 g/l, $CuSO_4 \cdot 5H_2O$ 6.0 g/l, $ZnSO_4 \cdot 7H_2O$ 20 g/l, $MnSO_4$ 3.0 g/l and $H_2SO_4$ 5.0 ml/l

The yeast extracts utilized in the present invention include but are not limited to yeast extracts selected from the group consisting of Amberex™ 1003 (Universal Foods Corporation) and Bacto™ Yeast Extract (Difco Laboratories Incorporated).

Trace metals utilized in the present invention are those trace metals generally utilized in yeast growth provided in an amount sufficient to not limit the growth rate or astaxanthin production of *Phaffia rhodozyma* which include but are not limited to trace metals selected from the group consisting of cobalt and molybdenum.

The fermentation temperature should generally range from about 18° C to about 22° C and preferably should be about 20° C.

The dissolved oxygen content in the fermentation vessel where the fermentation is conducted in a batch-fed manner may range from about 10% to about 80% of saturation and preferably will range from about 30% to about 60% of saturation. The dissolved oxygen content in a continuous fermentation should range from about 70% to about 100% of saturation and preferably be in the range of from about 70% to about 80% of saturation. The pH at which the *Phaffia rhodozyma* cells are cultivated should range from about 3.0 to about 5.5 and preferably the pH will range from about 4.5 to about 5.4.

After the fermentation broth containing the *Phaffia rhodozyma* cells has reached a desired cell density or astaxanthin content, the cell mass may be harvested. It is preferred that the *Phaffia rhodozyma* culture be held in a stationary phase for from the range of from about 4 to about 24 hours and most preferably in the range of from about 8 to about 12 hours to increase the astaxanthin yield.

However, *Phaffia rhodozyma* should not be maintained for extended periods of time in a stationary phase because the *Phaffia rhodozyma* cells will form thick cell walls which will be detrimental to cell breakage.

Cell Breakage

Salmonids, crustaceans and birds cannot utilize astaxanthin from unbroken *Phaffia rhodozyma* cells.

To utilize *Phaffia rhodozyma* as a dietary source of astaxanthin the cell walls of *Phaffia rhodozyma* must be disrupted by physical, chemical, mechanical, or enzymatic means. *Phaffia rhodozyma* cell walls are very resistant to normal lysis protocols. For example, bead milling will only release ~40% of the astaxanthin present in *Phaffia rhodozyma* cells after three passes through a bead mill (more passes through a bead mill will not substantially increase the release of astaxanthin). A Gaulin Press will release ~95% of the astaxanthin present in *Phaffia rhodozyma* but only after three passes through the Gaulin Press (which is time consuming and requires a significant capital expenditure). Enzymatic lysis of *Phaffia rhodozyma* also had not proven to be economical or effective in releasing astaxanthin from *Phaffia rhodozyma* until the discovery of the present invention.

Applicants have discovered that an effective amount of a digestive enzyme preparation from the fungus *Trichoderma harzianium* is capable of digesting the cell wall of *Phaffia rhodozyma*. This results in an almost complete availability of the astaxanthin present in *Phaffia rhodozyma* as determined by comparison to acetone extraction which is described in Example I.C.2.

Suitable strains of *Trichoderma harzianium* are publicly available from the American Type Culture Collection, 12301 Parklawn Drive, Rockville, Maryland (such as ATCC 64986). These strains may be cultured and stimulated to produce digestive enzymes by submerged culture fermentation as is known by those skilled in the art. One suitable source of *Trichoderma harzianium* digest enzyme preparations is Novo Enzymes SP-299-Mutanase.

*Phaffia rhodozyma* cells containing astaxanthin should be treated with an effective amount of *Trichoderma harzianium* digestive enzyme preparation to result in the availability of substantially all the astaxanthin contained therein. Generally the amount of digestive enzyme preparation per 100 grams/liter of aqueous *Phaffia rhodozyma* will be dependent on the temperature, pH and condition of the *Phaffia rhodozyma* cells employed. As a guideline it is recommended that the amount of *Trichoderma harzianium* utilized range from about 0.2 units to about 10.0 units of *Trichoderma harzianium* digestive enzyme preparation per 100 grams/liter of *Phaffia rhodozyma* cells. A unit is defined as the amount of *Tricoderma harzianium* digestive enzyme which will provide the equivalent amount of released astaxanthin as the acetone extraction described in Example I.C.2, on a sample of aqueous *Phaffia rhodozyma* with a density of 100 grams/liter, removed while in a logarithmic growth phase, when the digestive enzyme is contacted with the *Phaffia rhodozyma* cells at 22° C, and pH 4.5 and allowed to incubate for 24 hours.

Temperature at which *Phaffia rhodozyma* cells are contacted with the digestive enzyme preparation may be any temperature which allows the digestive enzyme preparation to digest *Phaffia rhodozyma* cell walls. Generally temperatures should range from about 0° C to about 60° C. Preferred for the practice of this invention are temperatures in the range of frcm about 20° C to about 30° C.

The pH at which *Phaffia rhodozyma* cells are contacted with the digestive enzyme preparation may be any suitable pH which permits the digestive enzyme preparation to digest *Phaffia rhodozyma* cell walls. Generally the pH at which *Phaffia rhodozyma* cells are contacted with the digest enzyme preparation should range of from about pH 4.0 to about pH 5.5 and preferably be in the range of from about pH 4.5 to about pH 5.0.

*Phaffia rhodozyma* cells containing astaxanthin may be contacted with the digestive enzyme preparation derived from *Trichoderma harzianium* at any time during the life cycle of *Phaffia rhodozyma*. However, it is preferred that the *Phaffia rhodozyma* cells be contacted with the digestive enzyme preparation as soon as possible after the *Phaffia rhodozyma* cells have been in a logarithmic growth phase, preferably in the range of from about 0 hours to about 72 hours after a logarithmic growth phase and most preferably in the range of from about 0 hours to about 24 hours.

The mixing of an aqueous suspension of *Phaffia rhodozyma* cells and the *Trichoderma harzianium* digestive enzyme preparation may be accomplished by any suitable means. Mixing is generally accomplished by contacting a dried digestive enzyme preparation with an aqueous *Phaffia rhodozyma* fermentation broth or aqueous cell suspension and admixing said dry digestive enzyme preparation into solution.

The digestive enzyme preparation derived from *Trichoderma harzianium* may be contacted with *Phaffia rhodozyma* cells which contain astaxanthin for an amount of time effective to result in the substantial release of astaxanthin present in the *Phaffia rhodoymza* cells as compared to acetone extraction described in Example I.C.2. The amount of time depends on the cell concentration, pH, temperature and units of digestive enzyme preparation utilized. Generally the time of contacting the *Phaffia rhodozyma* cells with the digestive enzyme preparation derived from *Trichoderma harzianium* should be in the range of about 12 hours to about 24 hours and preferably the time of contacting will be about 24 hours.

Drying of *Phaffia rhodozyma* Cells

The *Phaffia rhodozyma* cells after having been broken or digested in a manner which renders the astaxanthin contained therein available for use as a dietary pigment supplement can be dried. Drying may be performed using a fluidized bed drier, drum drier, or spray drier. Spray drying is presently preferred because of the short exposure time to high temperatures which could possibly oxidize the astaxanthin present.

After drying, the resultant product will be a powdery yeast material which may be recovered by any suitable means such as a cyclone, and further handled for use in feed, storage, or shipping.

## Examples

### Strains

| | | |
|---|---|---|
| *Phaffia rhodozyma* | PC 8055 | NRRL Y-10921 |
| *Phaffia rhodozyma* | PC 8089 | NRRL Y-18579 |
| *Phaffia rhodozyma* | PC 8109 | NRRL Y-18581 |
| *Phaffia rhodozyma* | PC 8108 | NRRL Y-18582 |
| *Phaffia rhodozyma* | PC 8107 | NRRL Y-18583 |

Media, Buffers and Solutions

| | | | |
|---|---|---|---|
| YEPD | 1% Bacto yeast extract | | |
| | 2% Bacto peptone | | |
| | 2% Dextrose | | |
| Citrate buffer | pH 5.5 | .1M | |
| Rich Assay Growth Medium | Bacto Yeast Extract | 9.0 | g/l |
| | Difco Malt Extract | 6.0 | g/l |
| | Dextrose | 10.0 | g/l |
| | $KH_2HPO_4$ | 15.0 | g/l |
| | $K_2HPO_4$ | 1.0 | g/l |
| | $MgSO_4 \cdot 7H_2O$ | 0.5 | g/l |
| | $CaSO_4 \cdot 2H_2O$ | 0.04 | g/l |
| | $(NH_4)_2SO_4$ | 3.0 | g/l |
| | $FeSO_4 \cdot 7H_2O$ | 16.25 | mg/l |
| | $CuSO_4 \cdot 5H_2O$ | 1.5 | mg/l |
| | $ZnSO_4 \cdot 7H_2O$ | 5.0 | mg/l |
| | $MnSO_4 \cdot H_2O$ | 0.75 | mg/l |
| | Biotin | 0.05 | mg/l |
| | Thiamine | 1.00 | mg/l |
| | Water | 1 L | |
| Modified YMA Medium | Bacto Yeast Extract | 3 | g/l |
| | Difco Malt Extract | 3 | g/l |
| | Dextrose | 20 | g/l |
| | Agar | 20 | g/l |
| | Water | 1.0 | L |

Example I

Development of Improved Strains of *Phaffia rhodozyma*

The following protocols were used to develop strains PC 8089, PC 8109, PC 8108, and PC 8107.

A. NTG Mutagenesis of *Phaffia rhodozyma*

*Phaffia rhodozyma* was subcultured into 50 ml of YEPD in a 250 ml Ehrlenmeyer flask and incubated on a shaker for 48 hr at 20 °C. The cells were then centrifuged in sterile 30 ml tubes at 12,000 g at 4 °C for

10 min and washed two times with 30 ml of sterile, pH 5.5 citrate buffer. The cell pellet was resuspended in 27 ml of sterile pH 5.5 citrate buffer and 3 ml of 1mg/ml NTG (in pH 5.5 citrate buffer) was added. The cell suspension was then incubated for 15 min at room temperature without shaking. The suspension was centrifuged at 10,000 rpm at 4° C for 5 min and then washed two times with sterile deionized water. The cells were then resuspended in 30 ml of YEPD in a 250 ml Ehrlenmeyer flask and incubated on a shaker for 20 min at 20° C. Using sterile 0.1M $MgSO_4$, the cells were diluted 1/100 and 0.1 ml was plated on 20 YEPD plates. These plates were then incubated at 20° C for ten days. After ten days the plates were counted and scored for mutants.

B. EMS Mutagenesis of *Phaffia rhodozyma*

*Phaffia rhodozyma* was subcultured into 50 ml of YEPD in a 250 ml Ehrlenmeyer flask and incubated on a shaker at 20° C for 2 days. The cells were centrifuged in sterile 30 ml tubes at 12,000 g at 4° C for 10 min. The pellet was then washed two times with 30 mls of sterile, pH 5.5 citrate buffer. 0.9 ml of the cell suspension was placed into each of 13 sterile 1.5 ml Eppendorf centrifuge tubes and 75 µl of EMS (ethylmethanesulfonate) was added to each tube. One tube was immediately removed, centrifuged with a microfuge to pellet the cells, washed two times with 1.0 ml of sterile deionized water, resuspended in 1.0 ml of sterile deionized water and kept on ice until time for dilution. This step was repeated every 5 min for subsequent tubes (up to 60 min total exposure to EMS). All tubes were then diluted $10^2$, $10^3$, $10^4$, $10^5$, $10^6$, and $10^7$ times in sterile 0.1M $MgSO_4$ and 0.1 ml of each dilution was plated on 2 YEPD plates. All plates were incubated at 20° C for 10 days. After 10 days the plates were counted and scored for mutants.

C. Screening Process

1. Plate Assay of Astaxanthin

Modified YMA plates were streaked with cultures to be tested and incubated at 20-22° C aerobically. After four days, using a sterile applicator stick or loop, one or two large colonies were scraped off (0.1-0.2 grams net weight) from each Modified YMA culture plate. The cells were resuspended in 1.0 ml of deionized, distilled water and placed in a 2.0 ml conical bottom Eppendorf microcentrifuge tube.

0.1 ml of each cell suspension was pipetted into 9.9 ml of deionized, distilled water, and the absorption was measured at 600 nm, to determine the cell concentration (in grams of washed cell dry weight per liter) with this method, an original cell concentration of 15 g/L has at $A_{600}$ (using a 1 cm light path) an absorption of 0.550. The measurement is linear over an absorption range of 0.000 to 1.000, or 0 to 27 gm/L of cells.

The original cell suspension (0.9 ml volume) was then pelleted by centrifugation at 10,000 g at 4° C and the supernatant was decanted. Acetone was added to provide a total volume of 1.0 ml. 0.25 grams of 450-500 micron glass beads were added and vortexed for at least 10 min. total time (with periodic cooling intervals on ice). After 10 minutes the vortexing should have resulted in the breakage of substantially all the *Phaffia rhodozyma* cells present, which should be verified microscopically. If the breakage was not substantially complete the bead milling process should be repeated until substantial breakage does occur. The resulting cell debris and glass beads were pelleted by centrifugation at 10,000 g and 4° C. 0.5 ml of supernatant was removed and 1.0 ml of acetone was added to the sample. The absorption was then measured at 478 nm.

Astaxanthin concentration was calculated as follows:

assume 1 µg/l of pure astaxanthin
has an $A_{478} = 160 \times 10^{-6}$

Total astaxanthin concentration of the original 1.0 ml cell suspension:

$$[\text{astaxanthin}] \text{ in } \mu g/l = \frac{A_{478}}{bX160} \times 10^6 \times \text{dilution factor}$$

where b = length of light path in cm (usually 1) and

$$\text{dilution factor} = \frac{3}{0.9}.$$

Cellular astaxanthin concentration is then calculated as follows:

$$[\text{astaxanthin}] \text{ in } \mu g/g \text{ of cells} = \frac{\text{astaxanthin concentration } (\mu g/l)}{\text{cell concentration } (g/l)}$$

2. Shake Flask Assay of Astaxanthin

50 ml of Rich Assay Growth Medium was inoculated with a loop full of *Phaffia* culture from a 7 day old plate culture in a 250 ml Ehrlenmeyer flask. The inoculated flask was then incubated at 20-22° C with vigorous shaking.

After five days each culture was harvested by centrifugation at 12,000 g for 10 min at 4° C. Each pellet was washed 2x with 100 ml of deionized, distilled water and finally resuspended in 100 ml of deionized, distilled water. 0.1 ml of each cell suspension was pelleted into 9.9 ml of deionized, distilled water and the absorption was measured at 600 nm to determine the cell concentration. Using this method, an original cell concentration of 15 g/l has at $A_{600}$ (using a 1 cm light path) an absorption of 0.550. The measurement is linear over an absorption range of 0.000 to 1.000 or 0 to 27 gm/l of cells.

10 ml of each original cell suspension was pelleted and resuspended in 1.0 ml of deionized, distilled water and transferred to a 2.0 ml conical bottom Eppendorf centrifuge microfuge tube. The cells were pelleted in the Eppendorf tube by centrifugation at 10,000 g and 4° C. The supernatant was then decanted and acetone was added to provide a 1.0 ml final volume.

0.25 grams of 450-500 micron glass beads were added to the sample and vortexed for at least 10 min total time (with periodic intervals for cooling on ice). After 10 min. the vortexing should have resulted in the breakage of substantially all the *Phaffia rhodozyma* cells present, which should be verified microscopically. If the breakage was not substantially complete the bead milling process should be repeated until substantial breakage did occur. The glass beads and resulting cell debris were pelleted by centrifugation at 1,000 g and 4° C. 0.5 ml of the supernatant was removed and 1.0 ml of acetone was added. The absorption was measured at 478 nm.

Assume 1 $\mu$g/1 of pure astaxanthin has an $A_{478} = 160 \times 10^{-6}$

Total astaxanthin concentration of the original 100 ml cell suspension:

$$[\text{astaxanthin}] \text{ in } \mu g/l = \frac{A_{478}}{bx160} \times 10^6 \times \text{dilution factor}$$

where b = length of light path in cm (usually) and dilution factor

$$= \frac{3}{1.0 \times 10}$$

Cellular astaxanthin concentration was then calculated as follows:

$$[\text{astaxanthin}] \text{ in } \mu g/g \text{ of cells} = \frac{\text{astaxanthin concentration } (\mu g/l)}{\text{cell concentrations } (g/l)}$$

or

$$\frac{\text{astaxanthin concentration } (\mu/l)}{\text{cell concentration } (g/l) \text{ DCW}}$$

DCW is the abbreviation for dry cell weight. The dry cell weights were determined by taking a 25 ml sample of the broth and centrifuging the broth until a pellet of cells was formed. The supernatent was poured off and the pellet was resuspended in deionized water to provide a solution with a final volume of 25 ml. The resuspended solution was again centrifuged until a pellet of cells was formed. The supernatent was poured off and the pellet of cells was mixed with enough deionized water to form a pourable cell slurry. The slurry was placed in an aluminum tare pan. The centrifuge tube was also rinsed to remove any cell residue with approximately 5 ml of deionized water which was also added to the pan containing the cell slurry. The pan was placed in a drying oven at 80°C for 12 hours. At the end of the 12 hours the pan was weighted and the tare weight subtracted to give the dry cell weight.

3. <u>HPLC Assay of Astaxanthin</u>

Five 0.2 ml samples of yeast broth containing 0.05 grams per ml of yeast as measured at 600 nm were separately placed in five 1.5 ml conical bottomed centrifuge tubes. Cell concentration was calculated using an original cell concentration of 15 grams per liter and having an absorption of 0.550 (using a 1 cm lightpath, assuming the measurement is linear between a 0 to 15 gram per liter concentration). The samples were then centrifuged in a microcentrifuge at 14,000 g for 5 minutes at 4°C to pellet the cells. 1.0 ml of acetone was added to each of the samples as well as 0.25 grams of glass beads (450-500 micron glass beads washed in 10% phosphoric acid rinsed in distilled dionized water and dried at 80°C). The tubes were then vortexed vigorously for 2 minutes at 4°C and centrifuged in the microcentrifuge at the 14,000 g for 10 minutes at 4°C to pellet the glass beads and cell debris. The supernatant was decanted from all the tubes into a 6 dram glass vial. The addition of acetone and vortexing followed by centrifuging were repeated until the supernatant produced by this process was colorless. The supernatant was then combined and dried in a hood in darkness under a nitrogen atmosphere at room temperature. The pigment which was left after the drying process was then mixed with exactly 5 mls of methanol and allowed to redissolve for 30 minutes. The methanol pigment solution was then filtered through a 0.2 micron filter. A 100 microliter sample of the pigment solution was then run in a Waters HPLC. The HPLC was run isocratically, with methanol as a solvent, a 2 ml per minute flow rate and the column was maintained at room temperature. The detector was set at 478 nm. The column was run for 10 minutes. The asaxthanin retention was approximately 2.6 to 2.7 minutes. Sample peak areas were calculated by a Hewlett Packard 3390A Integrater. A Waters radial-PAK C18 column (P-N 86342) was the column utilized in the HPLC.

Cellular astaxanthin concentration was then calculated as follows:

$$[\text{astaxanthin}] \text{ in } \mu g/g \text{ of cells} = \frac{\text{astaxanthin concentration } (\mu g/l)}{\text{cell concentrations } (g/l)}$$

$$[\text{astaxanthin}] \text{ in } \mu g/g \text{ DCW} = \frac{\text{astaxanthin concentration } (\mu g/l)}{\text{cell concentrations } (g/l) \text{ DCW}}$$

DCW is the abbreviation for dry cell weight. The dry cell weights were determined by taking a 25 ml sample of the broth and centrifuging the broth until a pellet of cells was formed. The supernatent was poured off and the pellet was resuspended in deionized water to provide a solution with a final volume of 25 ml. The resuspended solution was again centrifuged until a pellet of cells was formed. The supernatent was poured off and the pellet of cells was mixed with enough deionized water to form a pourable cell slurry. The slurry was placed in an aluminum tare pan. The centrifuge tube was also rinsed to remove any cell residue with approximately 5 ml of deionized water which was also added to the pan containing the cell slurry. The pan was placed in a drying oven at 80°C for 12 hours. At the end of the 12 hours the pan was weighted and the tare weight subtracted to give the dry cell weight.

The standard was obtained by dissolving 5 mg of crystalline astaxanthin in 100 ml of methanol. To calculate the exact concentration the absorption was measured at 478 nm. The extinction coefficient for a 1% solution at 1 cm was determined to be 2,350. The purity of the standard solution was verified by running HPLC analysis on several dilutions. Aliquots of the standard may be stored for up to 4 weeks in a -80°C freezer in the dark.

D. Development of Strains

*Phaffia rhodozyma* mutants were developed from the parent strain PC 8055 (NRRL Y-10921) using either NTG or EMS mutagenesis as detailed in Examples IA and IB. The following flow chart illustrates the genetic history of each mutant strain. The numbers on the left hand column indicate the generation. The letters EMS and NTG represent the type of mutagensis performed to derive the next listed generation. Each generation utilized the plate and shake flask screenings as well as the astaxanthin extraction with acetone to screen hundreds of progeny before the next round of mutagensis was performed.

## Flow Chart of Mutant Development

| | | | | | |
|---|---|---|---|---|---|
| 1 | | | PC 8055 | | |
| | | | NTG | | |
| 2 | | | PC 8056 | | |
| | | | NTG | | |
| 3 | | | PC 8057 | | |
| | | | NTG | | |
| 4 | EMS | | PC 8058 | | NTG |
| | | | NTG | | |
| 5 | PC 8059 | | PC 8065 | | PC 8062 |
| | | EMS | NTG | NTG | |
| 6 | | PC 8088 | PC 8081 | | |
| | | NTG | NTG | | |
| 7 | | PC 8089 | PC 8082 | PC 8067 | PC 8070 |
| | | NTG | | | NTG |
| 8 | | | | | PC 8071 |
| | | | | | NTG |
| 9 | PC 8091 | PC 8093 | | | PC 8075 |
| | | | | | NTG |
| 10 | | | | | PC 8077 |

### A. Novel Strains

The following tables demonstrate the high astaxanthin levels produced in the novel strains of the present invention.

**TABLE 5**

| | Astaxanthin µg/g | |
|---|---|---|
| Mutant | Plate[1] | Shake Flask[2] |
| PC 8089 | 2516 | 2727 |
| PC 8109 | 2567 | 5455 |
| PC 8108 | 2551 | 6578 |
| PC 8107 | 2494 | 8004 |

[1]Plate concentrations were determined as set forth in Example I.

[2]*Shake flask concentrations were determined as set forth in Example I.*

## Claims

1. A strain of *Phaffia rhodozyma* which produces in the range of from about 2700 μg/g to about 8025 μg/g of astaxanthin on a dry weight basis when cultivated under suitable conditions in a shake flask.

2. The strain of claim 1 wherein the strain produces in the range of from about 5500 μg/g to about 8025 μg/g of astaxanthin on a dry weight basis when cultivated under suitable conditions in a shake flask.

3. The strain of claim 1 wherein the strain produces from in the range of from about 6500 μg/g to about 8025 μg/g of astaxanthin on a dry weight basis when cultivated under suitable conditions in a shake flask.

4. *Phaffia rhodozyma* strain deposited with the NRRL designated as Y-18579.

5. *Phaffia rhodozyma* strain deposited with the NRRL designated as Y-18581.

6. *Phaffia rhodozyma* strain deposited with the NRRL designated as Y-18582.

7. *Phaffia rhodozyma* strain deposited with the NRRL designated as Y-18583.

8. A method for the production of astaxanthin comprising cultivating under suitable conditions *Phaffia rhodozyma* strain NRRL Y-18579.

9. A method for the production of astaxanthin comprising cultivating under suitable conditions *Phaffia rhodozyma* strain NRRL Y-18581.

10. A method for the production of astaxanthin comprising cultivating under suitable conditions *Phaffia rhodozyma* strain NRRL Y-18582.

11. A method for the production of astaxanthin comprising cultivating under suitable conditions *Phaffia rhodozyma* strain NRRL Y-18583.

European Patent Office

# EUROPEAN SEARCH REPORT

Application Number

EP 91 10 0682

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X,D,A | WO-A-8 808 025 (DANISCO BIOTEKNOLOGI A/S)<br>* page 16, lines 6 - 21; claim 16 *<br>- - - | 1,2-11 | C 12 P 23/00<br>C 12 N 1/145<br>C 12 R 1/645 |
| A | APPLIED AND ENVIRONMENTAL MICROBIOLOGY vol. 55, no. 1, January 1989, pages 116 - 124; AN, G.-H. et al.: "Isolation of Phaffia rhodozyma mutants with increased astaxanthin content"<br>* the whole document *<br>- - - - - | 1-11 | |
| | | | TECHNICAL FIELDS SEARCHED (Int. Cl.5) |
| | | | C 12 P<br>C 12 R |

The present search report has been drawn up for all claims

| Place of search | Date of completion of search | Examiner |
|---|---|---|
| The Hague | 12 April 91 | ANDRES S.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document